# EUROPÄISCHE PATENTSCHRIFT

(11) **EP 0 661 958 B1**
(45) Veröffentlichungstag und Bekanntmachung des Hinweises auf die Patenterteilung: **18.12.1996**
(21) Anmeldenummer: 93920730.4
(22) Anmeldetag: 15.09.1993
(51) Int. Cl.: A61F 13/02, B65C 1/02

(54) **TRANSFERVERFAHREN ZUR HERSTELLUNG TRANSDERMALER THERAPEUTISCHER SYSTEME**
TRANSFER PROCESS FOR MAKING TRANSDERMAL THERAPEUTIC SYSTEMS
PROCEDE DE TRANSFERT POUR LA FABRICATION DE SYSTEMES THERAPEUTIQUES TRANSDERMIQUES

(30) Priorität: 25.09.1992 DE 4232279
(43) Veröffentlichungstag der Anmeldung: 12.07.1995
(73) Patentinhaber: LTS LOHMANN Therapie-Systeme GmbH, 56567 Neuwied (DE)
(72) Erfinder: HILLE, Thomas, D-56564 Neuwied (DE); DEURER, Lothar, D-56077 Koblenz (DE); STEINBORN, Peter, D-56567 Neuwied (DE); GRADER, Ludwig, D-56626 Andernach (DE); ANHÄUSER, Dieter, D-56581 Melsbach (DE)
(74) Vertreter: Flaccus, Rolf-Dieter, Dr.
(86) Internationale Anmeldenummer: EP9302495
(87) Internationale Veröffentlichungsnummer: WO9407449

(56) Entgegenhaltungen:
- DE-A- 2 555 910
- DE-A- 3 233 546
- DE-A- 3 618 542

## Beschreibung

Die Erfindung betrifft ein Verfahren zur Herstellung transdermaler therapeutischer Systeme nach dem Oberbegriff des Patentanspruchs 1.

Durch die DE-PS 32 04 582 ist ein Verfahren zum kontinuierlichen Herstellen und Füllen von Pflasterverpackungen mit einem transdermal zu verabreichenden Wirkstoff bekannt. Dabei wird der Wirkstoff portionsweise in bestimmten Abständen auf eine Trägerfolie aufgebracht, mittels metallischer Deckfolienabschnitte abgedeckt und dann unter Verwendung einer diese umgebenden Zwischenlagefolie von einer elastischen, klebseitig gegen diese gerichtet einseitig klebefähigen Hauthaftfolie überdeckt und danach die Wirkstoffportionen mit den sie umgebenden Folienschichten in der gewünschten Größe ausgestanzt. Dieses Verfahren bedingt einen verhältnismäßig großen technischen Aufwand und ist mit verhältnismäßig großen Mengen überflüssigen Materials behaftet.

Es wurde auch schon ein Verfahren zur kontinuierlichen Herstellung transdermaler therapeutischer Pflaster vorgeschlagen, bei dem zunächst durch Beschichten einer Zwischenträgerfolie mit einer fließfähigen, wirkstoffhaltigen Zubereitung ein Laminat hergestellt und dieses dann zu Streifen vorgegebener Breite und in weiteren Verfahrensschritten zu wirkstoffhaltigen Abschnitten bestimmter Länge zurechtgeschnitten und diese schließlich in vorgegebenen Abständen auf eine sie allseitig überdeckende Schutzfolie aufgebracht und schließlich durch Durchtrennen der Schutzfolie quer zum Bandverlauf zwischen den wirkstoffhaltigen Abschnitten in Einzelpflaster unterteilt werden. Dieses Verfahren löst zwar sehr vorteilhaft die gestellte Aufgabe, den Wirkstoffverlust weitgehend zu unterbinden bzw. zu minimieren, sie ist jedoch wegen der vielen Verfahrensschritte verhältnismäßig umständlich.

Ziel der vorliegenden Anmeldung ist es daher, vereinzelte wirkstoffhaltige Abschnitte mit hoher Geschwindigkeit und großer Genauigkeit und ohne Wirkstoffverlust von einer ersten Bahn in genau vorbestimmten Abständen hintereinander auf eine die Abschnitte allseitig überragende zweite Bahn in technisch einfacher und zuverlässiger Weise zu übertragen. Diese Aufgabe wird erfindungsgemäß mit einem Verfahren gemäß dem Kennzeichen des Patentanspruchs 1 gelöst. Zweckmäßige weitere Ausgestaltungen der Erfindung sind in den Unteransprüchen beschrieben.

Durch das Aufbringen der wirkstoffhaltigen Abschnitte von der ersten Bahn auf die zweite während einer Ruhephase der Bahnen entfällt das Erfordernis, die Geschwindigkeit der Bahnen in ihrer Bewegungsphase genau synchronisieren zu müssen und wird es möglich, die erforderlichen Weiterbewegungs- bzw. Vorzugslängen der Bahn in einfacher Weise genau einzustellen, wobei durch eine entsprechende größere Vorzugslänge der zweiten Bahn durch unterschiedliche Bewegungs- bzw. Ruhephasen und/oder Schnittlänge und/oder Geschwindigkeiten der beiden Bahnen sich die Abstände zwischen den wirkstoffhaltigen Abschnitten in der gewünschten Weise leicht variieren lassen.

Der Vorteil ist die Verwendung einer an sich vom Aufbringen von Etiketten auf Gegenständen in Etikettiervorrichtungen her bekannten Transfereinrichtungen mit sogenannter Spenderkante, wie sie beispielsweise aus der DE-OS 15 11 873, der DE-PS 25 55 910, der DE-OS 32 33 546, der DE-PS 36 18 542 und dem DE-GM 83 21 825 bekannt sind. Durch die Verwendung einer eigenbeweglichen Übergabe- bzw. Transfereinrichtung ist es dabei insbesondere möglich, hohe Produktionsgeschwindigkeiten zu realisieren. An der Spenderkante werden die wirkstoffhaltigen Abschnitte zunächst von der Trägerfolie der ersten Bahn gelöst und unmittelbar danach auf die zweite Bahn aufgelegt. Mittels einer Kaschierrolle kann diese Übertragung auch in der umgekehrten Reihenfolge durchgeführt werden, d.h. die erste Bahn wird in ihrem ursprünglichen Verbund zunächst auf die zweite Bahn aufkaschiert und unmittelbar danach die Trägefolie der ersten Bahn wieder abgezogen. Geschieht diese Kaschierung abschnittsweise in der Ruhephase der Bahnen durch eine Eigenbewegung der Kaschierrolle, ist es möglich, hohe Produktionsgeschwindigkeiten zu realisieren. Dadurch, daß das Durchtrennen der wirkstoffhaltigen Schicht sowie das Übertragen der dabei gebildeten wirkstoffhaltigen Abschnitte in vorgegebenen Abständen unmittelbar hintereinander "on-line" auf ein und derselben Vorrichtung vorgenommen werden, wird vorteilhaft die Ausbildung von Klebstoffbrücken sicher verhindert.

Bei der Durchführung des Verfahrens kann beispielsweise so vorgegangen werden, daß die wirkstoffhaltige Schicht in dem ersten bahnförmigen Laminat zwischen einer Hilfsschicht und einer klebstoffabweisend ausgerüsteten, beispielsweise silikonisierten Folie angeordnet wird. Die wirkstoffhaltigen Abschnitte bestehen dann aus der selbstklebenden wirkstoffhaltigen Schicht und der Hilfsschicht und werden durch die Übergabe- bzw. Transfervorrichtung so auf die klebstoffabweisend ausgerüstete zweite Bahn übertragen, daß die wirkstoffhaltige Schicht direkt auf der klebstoffabweisen ausgerüsteten Folie liegt. Als Transfereinrichtung kommt dabei in vorteilhafter Weise die bewegliche Spenderkante zum Einsatz.

Eine andere Möglichkeit zur Gestaltung des Verfahrens besteht beispielsweise darin, daß die zweite Bahn selbstklebend beschichtet ist. Die wirkstoffhaltigen Abschnitte, die wiederum aus der wirkstoffhaltigen Schicht und einer Hilfsschicht bestehen, werden so auf die zweite Bahn übertragen, daß die Hilfsschicht direkt auf der selbstklebenden zweiten Bahn liegt. Als Transfereinrichtung können sowohl die bewegliche Spenderkante als auch die bewegliche Kaschierrolle verwendet werden. Weitere Möglichkeiten der Gestaltung des Verfahrens bestehen darin, daß die wirkstoffhaltige Schicht in dem ersten bahnförmigen Laminat als selbstklebende Schicht auf einer klebstoffabweisend ausgerüsteten Trägerfolie vorgesehen wird. Die wirkstoffhaltigen Abschnitte bestehen dann nur aus der selbstklebenden Schicht und werden durch die Übergabe- bzw. Transfervorrichtung auf die zweite Bahn übertragen. Stattdessen kann die wirkstoffhaltige Schicht in dem ersten bahnförmigen Laminat auch als nicht selbstklebende Schicht auf einer Trägerfolie angeordnet werden. Die wirkstoffhaltigen Abschnitte bestehen dann nur aus der nicht selbstklebenden Schicht und werden durch die Übergabe- bzw. Transfervorrichtung auf eine zweite selbstklebend beschichtete Bahn übertragen. Es kann aber auch vorgesehen werden, die wirkstoffhaltige Schicht in der ersten Bahn als Laminat aus drei oder mehr Schichten auf einer Trägerfolie anzuordnen. Die wirkstoffhaltigen Abschnitte werden dann durch die Übergabe- bzw. Transfervorrichtung in der einen oder anderen Reihenfolge der aufeinanderfolgenden Schichten auf die zweite Bahn übertragen.

Wird bei dem erfindungsgemäßen Verfahren für den schrittweisen Transport der ersten Bahn ein Zangenvorzug eingesetzt, so bestimmt dieser die Länge der wirkstoffhaltigen Abschnitte. Die Bewegung der Übergabe- bzw. Transfervorrichtung kann mit der Bewegung des Zangenvorzugs synchronisiert werden. Auf einfache und zuverlässige Weise kann das beispielsweise durch eine direkte mechanische Verbindung erreicht werden. Dann entfallen aufwendige Steuerungsmaßnahmen und jeder wirkstoffhaltige Abschnitt wird exakt in seiner vollen Länge übergeben. Das Ausmaß der Bewegung der Übergabe- bzw. Transfervorrichtung wird dabei durch die Größe der zu übertragenden Abschnitte bestimmt. Anstelle eines Zangenvorzuges können selbstverständlich auch andere Mechanismen vorgesehen werden, z.B. ein Rollenvorzug oder dergleichen.

Sofern die Übergabe- bzw. Transfervorrichtung zusätzlich zu ihrer Bewegung in Bahnrichtung eine Bewegung senkrecht zur Bahnführung ausführen kann, so hat dies den Vorteil, daß die wirkstoffhaltigen Abschnitte während oder nach ihrer Übergabe auf die zweite Bahn an diese angedrückt werden können, insbesondere dann, wenn die wirkstoffhaltigen Abschnitte unterseitig an der Trägerfolie der ersten Bahn angeordnet sind.

Das Abziehen der delaminierten ersten Trägerfolie an der beweglichen Transfervorrichtung kann in vorteilhafter Weise so geschehen, daß während der Hin- und Herbewegung der Transfervorrichtung die Folie abgezogen wird. Dazu ist eine erste Umlenkrolle unmittelbar an der Transfervorrichtung befestigt und desweiteren eine zweite feststehende Umlenkrolle vorgesehen. Die Rollen sind dabei so angeordnet, daß die Trägerfolie an der Transfervorrichtung um einen Winkel von etwa 135 bis 180 Grad und von der ersten Umlenkrolle in entgegengesetztem Sinn im Mittel um etwa 120 Grad abgelenkt wird. Der Winkel in der vorderen Stellung der Übergabe- bzw. Transfervorrichtung soll dabei nicht weniger als etwa 100 Grad und in der hinteren Stellung nicht mehr als etwa 140 Grad befragen. Durch diese Anordnung der Umlenkrollen zum Abziehen der ersten Trägerfolie an der Übergabe- bzw. Transfervorrichtung kann ein ruhiger und spannungsarmer Lauf der Maschine erreicht werden.

Das erfindungsgemäße Verfahren kann auch so durchgeführt werden, daß die wirkstoffhaltige Schicht allein oder gemeinsam mit einer oder mehreren Hilfsschichten auf eine nicht klebende oder selbstklebende Bahn übertragen wird. Ein wesentlicher Vorteil ergibt sich dabei, wenn die Übergabe- bzw. Transfervorrichtung direkt mechanisch mit dem Zangenvorzug der ersten Bahn gekoppelt wird. Dann entspricht die Hublänge der Übergabe- bzw. Transfervorrichtung immer der Länge der zu übertragenden Abschnitte und es entfallen alle Steuerungsprobleme.

Die Erfindung ist in der Zeichnung in Ausführungsbeispielen gezeigt und wird anhand dieser im Folgenden erläutert. Es zeigen:
Figuren 1 bis 3 verschiedene Phasen einer Durchführungsform des erfindungsgemäßen Verfahrens und
Figur 4 eine andere Durchführungsform.

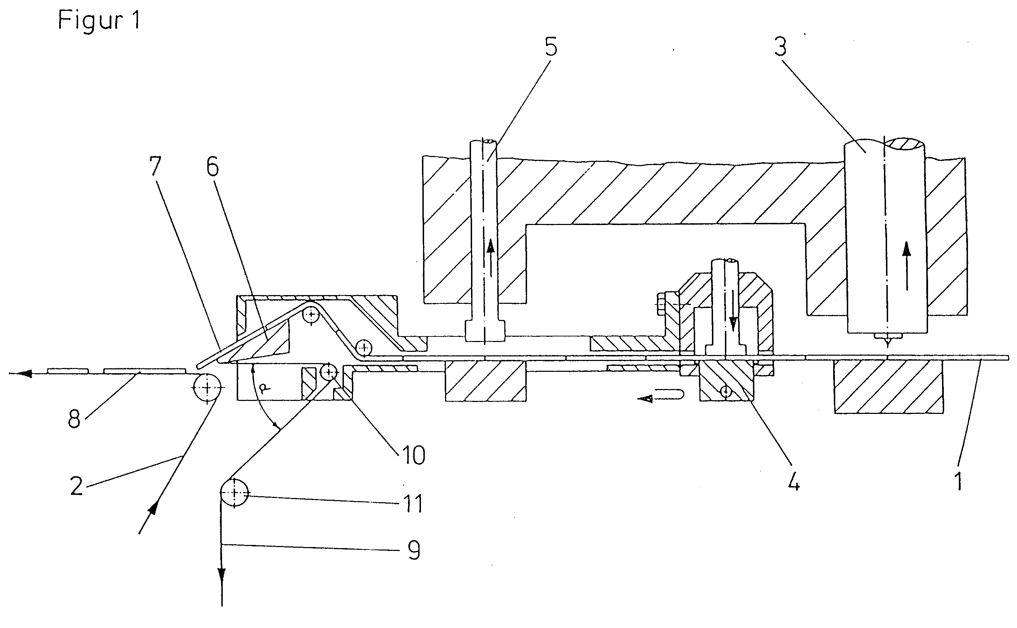

In Figur 1 bezeichnet 1 das erste bahnförmige Laminat bzw. die erste Bahn, wobei in nicht gezeigter Weise von unten nach oben gesehen beispielsweise aus einer beidseitig silikonisierten 50 µm dicken PET-Folie, die als Trägerfolie dient, einer wirkstoffhaltigen Schicht mit einem Flächengewicht von 100 g/m², die zu 57% ein Lösungspolyacrylat als Haftkleber, zu 25% einen Weichmacher, zu 10% Polymethacrylat und zu 8% den Wirkstoff Physostigminbase enthält, und einer 25 µm dicken PET-Hilfsfolie bestehen und 35 mm breit sein kann. Die zweite Bahn 2 ist 56 mm breit und wird zunächst nur durch eine beispielsweise 100 µm starke, beidseitig silikonisierte PET-Folie gebildet. Andere Schichtaufbauten bzw. Schichtfolgen sind selbstverständlich in vielerlei Varianten denkbar und möglich.

Mit der Stanze bzw. Schneidvorrichtung 3 werden die Hilfsfolie und die wirkstoffhaltige Schicht senkrecht zur Bahnrichtung mit einem geraden Schnitt durchtrennt, so daß quadratische Abschnitte mit den Abmessungen 35 mm x 35 mm entstehen. Die Trägerfolie wird dabei nicht durchtrennt, das heißt sie bleibt unversehrt. Mit Hilfe der beweglichen Zange 4 des Zangenvorzuges, anstelle dessen auch ein Rollenvorzug oder dergleichen vorgesehen sein könnten, wird die erste Bahn 1 in der Bewegungsphase von rechts nach links transportiert und mit Hilfe des Niederhalters 5 dann in der darauffolgenden Ruhephase festgehalten. An der Spenderkante 6 der Transfervorrichtung werden die wirkstoffhaltigen Abschnitte 7 von der Trägerfolie 9 gelöst. Die Bewegbarkeit der Bahnen sowie den Vorrichtungsteilen sind jeweils durch Pfeile bzw. Doppelpfeile angedeutet.

Die Figur 1 zeigt die Spenderkante in ihrem hinteren Totpunkt. Der Abschnitt 8 hat sich von der Spenderkante gelöst und ist vollständig auf die zweite Bahn 2 aufgelegt worden. Die Bahnen 1,2 sind noch in der Ruhephase, jedoch hat sich der Niederhalter 5 schon geöffnet und der Zangenvorzug 4 geschlossen.

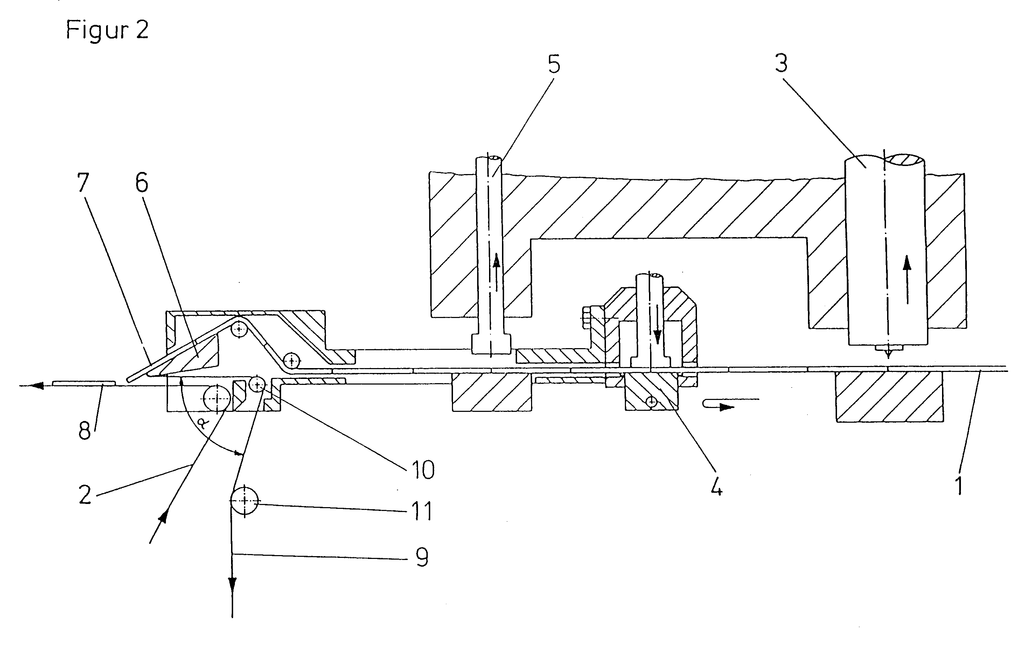

In Figur 2 ist der vordere Totpunkt erreicht. Der Zangenvorzug 4, die Spenderkante 6 und die Bahn 1 sind um 35 mm vorgezogen worden. Gleichzeitig ist die Bahn 2 um 56 mm vorgezogen worden, so daß sich zwischen den wirkstoffhaltigen Abschnitten 7 und 8, wie dargestellt, ein Zwischenraum von 21 mm gebildet hat.

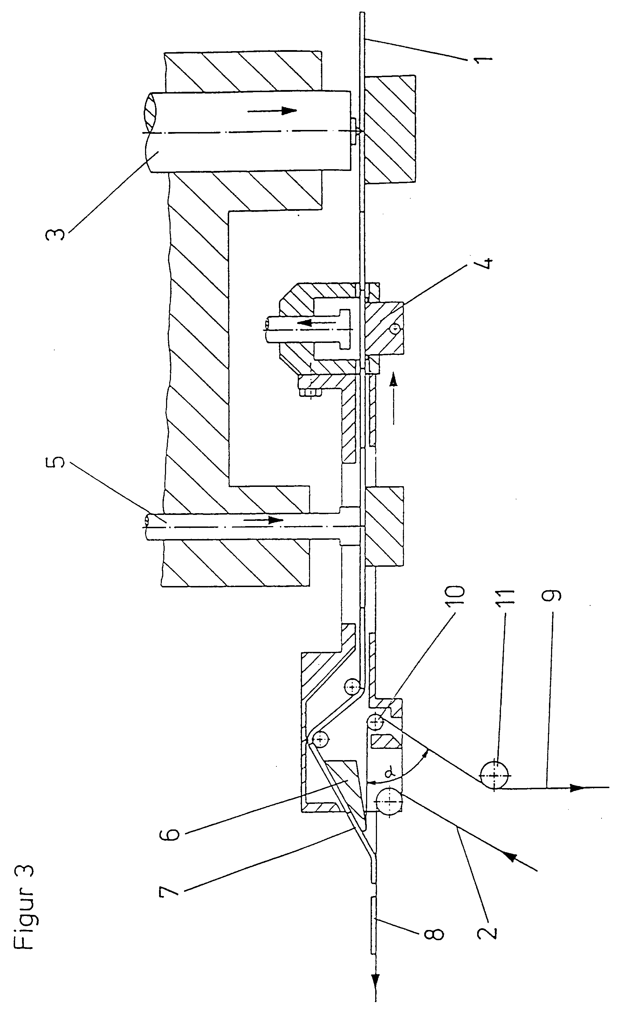

In Figur 3 schließlich sind die Bahnen 1 und 2 in Ruhe und die Stanze 3 vollzieht gerade das Durchtrennen. Der geöffnete Zangenvorzug 4 und die Spenderkante 6 bewegen sich gemeinsam rückwärts, wobei der wirkstoffhaltige Abschnitt 7 auf die zweite Bahn aufgelegt wird. Die Trägerfolie 9 wird dabei abgezogen. Wie aus den Figuren 1 bis 3 zu ersehen ist, erfolgt das Durchtrennen der wirkstoffhaltigen Schicht sowie das Übertragen der wirkstoffhaltigen Abschnitte im wesentlichen unmittelbar hintereinander. Die Spenderkante 6 ist mechanisch mit dem Zangenvorzug 4 verbunden, so daß sie die gleiche Bewegung ausführen. Die Trägerfolie 9 läuft über die mit der Spenderkante 6 bewegte Umlenkrolle 10 sowie über die feststehende Umlenkrolle 11. Von der Spenderkante 6 wird die Trägerfolie hier beispielsweise uni etwa 155 Grad abgelenkt und von der beweglichen Umlenkrolle 10 um einen Winkel von 180 Grad -α, der zwischen etwa 120 Grad in der vorderen Stellung (Figur 2) und etwa 135 Grad in der hinteren Stellung der Spenderkante (Figur 1) liegt.

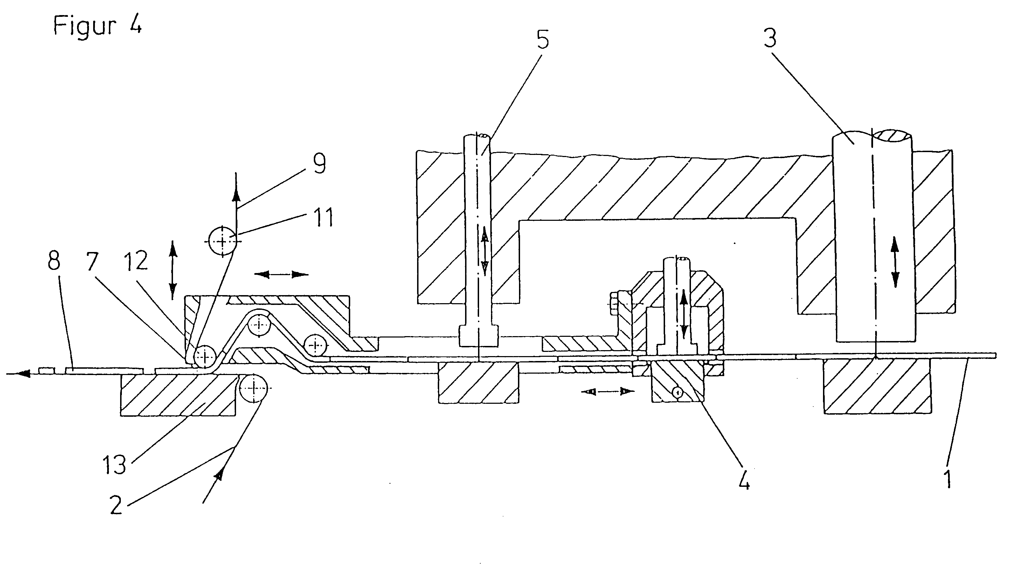

Bei der Durchführungsform des erfindungsgemäßen Verfahrens gemäß Figur 4 werden die wirkstoffhaltigen Abschnitte erst auf die zweite Bahn 2 aufgelegt und dann von der Trägerfolie getrennt. Das erste bahnförmige Laminat 1 weist eine unten liegende wirkstoffhaltige Schicht und eine oben liegende Trägerfolie auf. Die zweite Bahn 2 besteht aus einer selbstklebend beschichteten Folie. Mit der Stanze 3 wird die unten liegende wirkstoffhaltige Schicht mit einem geraden Schnitt senkrecht zur Bahnrichtung durchtrennt. Als Übergabe- bzw. Transfervorrichtung dient die Kaschierwalze 12, die synchron mit dem Zangenvorzug 4 , wie mit dem Doppelpfeil angedeutet ist, vor und zurück bewegt wird. Zusätzlich führt sie, wie ebenfalls mit Doppelpfeil angedeutet, noch eine Bewegung senkrecht zu den Bahnen aus, so daß sie während des Vorwärtshubes frei läuft und während des Rückwärtshubes die beiden Bahnen auf dem Kaschiertisch 13 andrückt. Dadurch wird der wirkstoffhaltige Abschnitt 7 auf die selbstklebende Bahn 2 laminiert und direkt danach wird die Trägerfolie 9 über die feststehende Rolle 11 abgezogen.

Bei der Herstellung des erfindungsgemäßen Pflasters kann je nach Bedarf, d.h. je nachdem, wofür das Pflaster Verwendung finden soll, eine mehr oder weniger große Anzahl von Schichten bzw. Hilfs- und/oder Schutzschichten vorgesehen werden, wobei für die einzelnen Schichten selbstverständlich jeweils geeignete Materialien, wie Metalle, vorzugsweise Aluminium, Polymere oder auch Textilien in Betracht kommen. Die einzelnen Schichten sind dabei entsprechend ihrem jeweiligen Zweck bzw. der von ihnen zu erfüllenden Aufgabe selbstklebend oder auch klebstoffabweisend, wirkstoffdurchlässig oder auch wirkstoffundurchlässig, flexibel oder nicht flexibel ausgebildet.

Von Bedeutung ist es in jedem Falle, daß die wirkstoffhaltigen Abschnitte 7,8 der Bahn 1, die selbstverständlich jede gewünschte Form wie rechteckig, quadratisch, oval, kreisrund oder dergleichen aufweisen können, im Hinblick auf die Vermeidung von Wirkstoffverlusten jedoch bevorzugt rechteckig oder quadratische Form aufweisen sollte, bis zu ihrer Übergabe an die Bahn 2 von der nicht unterbrochenen Trägerfolie bzw. -schicht 9 weitertransportiert werden, daß also die Bahn 1 beim Ausbilden der wirkstoffhaltigen Abschnitte 7,8 nicht schräg durchtrennt wird.

Die Haftkleberschicht kann beispielsweise aus einer Polymermatrix mit einem Grundpolymer und ggf. den üblichen Zusätzen hergestellt sein. Geeignete Polymere sind z.B. Silicone, Kautschuk, kautschukähnliche synthetische Homo-, Co- oder Blockpolymere, Polyacrylsäureester und deren Copolymere und Ester von hydriertem Kolophonium. Grundsätzlich kommen alle Polymere in Frage, die bei der Herstellung von Haftklebern eingesetzt werden und physiologisch unbedenklich sind. Besonders bevorzugt sind solche, die als Blockcopolymere auf Basis von Styrol und 1,3-Dienen, Polyisobutylenen oder Polymeren und Copolymeren aus Acrylat und/oder Methacrylat bestehen. Von den Blockcopolymeren auf Basis von Styrol und 1,3-Dienen finden bevorzugt lineare Styrol-Isopren-Styrol-Blockcopolymere Verwendung.

Die Wirkstoffreservoirschicht kann aus einer selbstklebenden Polymermatrix und dem Wirkstoff hergestellt sein, wobei die Polymermatrix aus einem Grundstoff und ggf. den üblichen Zusätzen hergestellt sein kann. Die Auswahl des Grundpolymers richtet sich nach den chemischen und physikalischen Eigenschaften des Wirkstoffs. Die Polymere können aus der gleichen Gruppe wie die wirkstofffreie Kleberschicht ausgewählt werden.

Als Wirkstoffe werden Substanzen verwendet, die ohne oder mit Resorptionsvermittler auf der Haut appliziert und eine lokale oder systemische Wirkung hervorrufen.

Stoffe mit lokaler Wirkung sind z.B. Antitranspirantia, Fungizide, Bacterizide und Bacteriostatica.

Stoffe mit systemischer Wirkung sind beispielsweise Antibiotika, Hormone, Antipyretica, Antidiabetica, Koronardilatatoren, herzwirksame Glycoside, Spasmolytica, Antihypertonica` Psychopharmaka, Migränemittel, Corticoide, Analgetica, Antikontrazeptiva, Antirheumatica, Anticholinergica, Sympatolytica, Sympatomimetica, Vasodilatatoren, Anticoagulantien und Antiarrhythmica.

Von dem verwandten Polymer und dem Wirkstoff abhängige mögliche Zusätze sind Weichmacher, Klebrigmacher, Stabilisatoren, Trägerstoffe, diffusions- und penetrationsregulierende Zusätze oder Füllstoffe. Die in Frage kommenden physiologisch unbedenklichen Substanzen sind bekannt. Die Eigenklebrigkeit der Reservoirschicht soll einen dauernden Kontakt zur Haut sicherstellen. Wie erwähnt, muß die Klebkraft der Reservoirschicht allein ausreichen, um die sichere Haftung der Reservoirschicht zu gewährleisten, da sie die Voraussetzung ist für eine ausreichende Wirkstoffabgabe des Systems. Sie kann durch den wirkstofffreien Haftkleberrand nicht kompensiert werden.

Die vor der Anwendung abzulösende Schutzschicht der Reservoirschicht kann beispielsweise aus denselben Materialien wie die zur Herstellung der Rückschicht verwendeten bestehen. Diese müssen jedoch - beispielsweise durch eine Siliconbehandlung - ablösbar gemacht werden. Andere ablösbare Schutzschichten sind beispielsweise Tetrafluorethylen, behandeltes Papier, Cellophan, Polyvinylchlorid u.ä.

Sofern eine Sperrschicht vorgesehen ist, die während der Lagerung der Systeme die Diffusion des Wirkstoffes oder der Weichmacher aus dem Wirkstoffreservoir in die wirkstofffreien Schichten verhindern soll, kann diese beispielsweise aus dem gleichen Material hergestellt sein wie eine ebenfalls vorgesehen Rückschicht.

Die Erfindung wird nachstehend an Ausführungsbeispielen näher erläutert.

### Beispiel 1:

In Figur 1 ist die Bahn ein 35 mm breites bahnförmiges Laminat, das, von unten nach oben gesehen, aus einer silikonisierten 100 µm dicken PET-Folie, die als Trägerfolie dient, einer 80 g/m² starken wirkstoffhaltigen Schicht, die zu 75% ein Lösungspolyacrylat als Haftkleber, zu 15% einen Weichmacher und zu 10% den Wirkstoff Physostigminbase enthält, und einer 15 µm dicken PET-Hilfsfolie hergestellt. Die zweite Bahn 2 hat eine Breite von 56 mm und besteht zunächst nur aus einer 100 µm starken silikonisierten PET-Folie. Mit der Stanze 3 werden die Hilfsfolie und die wirkstoffhaltige Schicht senkrecht zur Bahnrichtung mit einem geraden Schnitt durchtrennt, so daß quadratische Abschnitte mit den Maßen 35 mm x 35 mm entstehen. Die Trägerfolie bleibt unversehrt. Mit Hilfe der beweglichen Zange 4 des Zangenvorzugs wird das bahnförmige, wirkstoffhaltige Laminat in der Bewegungsphase von rechts nach links transportiert und mit Hilfe des Niederhalters 5 in der Ruhephase festgehalten. An der als Spenderkante 6 ausgebildeten Transfervorrichtung werden die wirkstoffhaltigen Abschnitte 7 von der Trägerfolie 9 gelöst. Figur 1 zeigt die Spenderkante in ihrem hinteren Totpunkt. Der Abschnitt 8 hat sich von der Spenderkante gelöst und ist vollständig auf die zweite Bahn aufgelegt worden. Die Bahnen sind noch in der Ruhephase, jedoch hat sich der Niederhalter 5 schon geöffnet und der Zangenvorzug 4 geschlossen. In Figur 2 ist der vordere Totpunkt erreicht. Der Zangenvorzug 4, die Spenderkante 6 und die Bahn 1 sind um eine Abschnittlänge vorgezogen worden.

### Beispiel 2:

Das erste bahnförmige Laminat 1 weist eine unten liegende 50 mm breite wirkstoffhaltige Schicht mit einem Flächengewicht von 100 g/m², die zu 30% ein Lösungspolyacrylat, zu 30% einen Weichmacher, zu 32% ein Polyvinylharz und zu 8% den Wirkstoff Physostigminbase enthält, und eine oben liegende 100 µm starke PET-Folie als Trägerfolie auf. Die wirkstoffhaltige Beschichtung ist schwach klebend. Alle mit ihr in Berührung kommenden Teile der Vorrichtung sind klebstoffabweisend beschichtet. Die zweite Bahn 2 ist 72 mm breit und ist aus einer 15 µm starken, selbstklebend beschichteten PET-Folie hergestellt. Mit der Stanze 3 wird die unten liegende Wirkstoffschicht durchtrennt, so daß quadratische Abschnitte mit den Maßen 50 mm x 50 mm entstehen. Die Trägerfolie bleibt unversehrt. Mit der Hilfe der beweglichen Zange 4 des Zangenvorzugs wird das bahnförmige wirkstoffhaltige Laminat in der Bewegungsphase von rechts nach links transportiert und mit Hilfe des Niederhalters 5 in der Ruhephase festgehalten. Als Transfervorrichtung dient die Kaschierwalze 12, die synchron mit dem Zangenvorzug 4 vor und zurück bewegt wird. Zusätzlich führt sie noch eine Bewegung in der Senkrechten aus, so daß sie während des Vorwärtshubes frei läuft und während des Rückwärtshubes die beiden Bahnen auf dem Kaschiertisch 13 andrückt. Die letztere Phase ist in Figur 4 dargestellt. Der wirkstoffhaltige Abschnitt 7 wird von der Kaschierwalze 12 auf die zweite Bahn 2 auflaminiert und danach die Trägerfolie 9 der ersten Bahn 1 über die Umlenkrolle 11 abgezogen. Da die Bahn 2 um 72 mm vorgezogen wurde, hat sich zu dem vorher übergebenen wirkstoffhaltigen Abschnitt 7 ein Zwischenraum von 22 mm gebildet.

Es ist zu erkennen, daß das Durchtrennen der wirkstoffhaltigen Schicht und das Übertragen der wirkstoffhaltigen Abschnitte gleichzeitig bzw. unmittelbar nacheinander erfolgen. Die Kaschierwalze 12 ist mechanisch mit dem Zangenvorzug 4 verbunden, so daß sie die gleiche Bewegung ausführt.

Es kann ein ruhiger Lauf der Maschine erreicht werden. Die wirkstoffhaltigen Abschnitte werden trotz der geringen Dicke der Hilfsschicht faltenfrei aufgelegt. Es gibt keinerlei Probleme mit Klebstoffbrücken. Es können Taktzahlen bis 6000 Stück/h erreicht werden.

## Patentansprüche

1. Verfahren zur Herstellung transdermaler therapeutischer Systeme, wobei die wirkstoffhaltige Schicht zunächst in Form eines Laminats mit einer Trägerschicht und gegebenenfalls weiteren Hilfs- und/oder Schutzschichten oder dergleichen als streifenförmige Bahn vorliegt und dann für sich allein oder in Verbindung mit wenigstens einer weiteren Schicht abschnittweise mittig auf eine zweite breitere Bahn übertragen wird, dadurch gekennzeichnet, daß die streifenförmige Bahn (1) und die zweite Bahn (2) mit unterschiedlicher Bewegungs- und Ruhephase und/oder unterschiedlicher Schrittlänge und/oder unterschiedlicher Geschwindigkeit schrittweise vorwärtsbewegt werden und daß jeweils in der Ruhephase der Bahnen (1,2) taktweise in derselben Vorrichtung das abschnittweise Unterteilen der wirkstoffhaltigen Schicht und gegebenenfalls einer oder mehrerer weiterer Schichten mit Ausnahme der Trägerschicht quer zur Bahnrichtung in anwendergroße wirkstoffhaltige Abschnitte (7,8) sowie das Übertragen auf die zweite Bahn unter Abzug der Trägerfolie (9) in vorzugsweise gleichen Abständen mitttels einer in Bahnrichtung hin- und herbeweglichen Übergabeeinrichtung vorgenommen wird.

2. Verfahren nach Anspruch 1, dadurch gekennzeichnet, daß die Übertragung der wirkstoffhaltigen Abschnitte (7,8) auf die zweite Bahn (2) während einer Ruhephase der Bahnen durch die Eigenbewegung einer an sich bekannten Transfereinrichtung mit Spenderkante (6) vorgenommen wird.

3. Verfahren nach Anspruch 1, dadurch gekennzeichnet, daß die Übertragung der wirkstoffhaltigen Abschnitte (7,8) auf die zweite Bahn (2) während einer Ruhephase der Bahnen (1,2) durch die Eigenbewegung einer als Kaschiervorrichtung mit Kaschierrolle (12) und Kaschiertisch (13) ausgebildeten Transfervorrichtung vorgenommen wird.

4. Verfahren nach Anspruch 2, dadurch gekennzeichnet, daß die wirkstoffhaltige Schicht in der streifenförmigen Bahn (1) als selbstklebende Schicht zwischen einer Hilfsschicht und einer klebstoffabweisend ausgerüsteten Folie angeordnet wird und die aus der wirkstoffhaltigen Schicht und der Hilfsschicht gebildeten wirkstoffhaltigen Abschnitte (7) mittels der Transfereinrichtung auf die als klebstoffabweisende Folie ausgebildete zweite Bahn (2) so übertragen werden, daß die wirkstoffhaltige Schicht direkt auf der klebstoffabweisend ausgerüsteten Folie liegt.

5. Verfahren nach Anspruch 2 oder 3, dadurch gekennzeichnet, daß die wirkstoffhaltige Schicht in der streifenförmigen Bahn als selbstklebende oder nichtklebende Schicht zwischen einer Hilfsschicht und einer abweisend ausgerüsteten Trägerschicht angeordnet wird und die aus der wirkstoffhaltigen Schicht und der Hilfsschicht gebildeten wirkstoffhaltigen Abschnitte mittels der Transfereinrichtung auf die selbstklebend beschichtete zweite Bahn so übertragen werden, daß die Hilfsschicht direkt auf der selbstklebenden Folie liegt.

6. Verfahren nach einem der Ansprüche 1 bis 3, dadurch gekennzeichnet, daß die wirkstoffhaltige Schicht in der streifenförmigen Bahn (1) als Laminat aus drei oder mehr Schichten auf einer Trägerfolie vorliegt und die wirkstoffhaltigen Abschnitte (7) in gleicher oder umgekehrter Reihenfolge der Schichten auf die zweite Bahn (2) übertragen werden.

7. Verfahren nach einem der Ansprüche 1 bis 6, dadurch gekennzeichnet, daß die wirkstoffhaltige Schicht auf der Trägerfolie als nichtklebende Schicht aufgebracht wird.

8. Verfahren nach einem der Ansprüche 1 bis 7, dadurch gekennzeichnet, daß die Bewegung der Transfereinrichtung und die Bewegung einer zangenartigen Einrichtung (4) zur Vorwärtsbewegung der ersten Bahn (1) miteinander gekoppelt sind.

9. Verfahren nach einem der Ansprüche 1 bis 8, dadurch gekennzeichnet, daß die Transfereinrichtung zusätzlich zu ihrer Bewegung in Bahnrichtung senkrecht zur Bahnrichtung bewegt wird und in der Ruhephase der Bahnen (1,2) gegen die zweite Bahn (2) angedrückt wird.

10. Vorrichtung zur Durchführung des Verfahrens nach einem der Ansprüche 1 bis 9, dadurch gekennzeichnet, daß zum Abziehen der delaminierten Trägerfolie (9) der ersten Bahn (1) eine mit der Transfereinrichtung bewegbare erste Umlenk- bzw. Kaschierrolle (10 bzw. 12) und eine feststehende zweite Umlenkrolle (11) angeordnet sind, und daß die abgezogene Trägerfolie (9) an der Transfereinrichtung zunächst um einen Winkel von etwa 135 bis 180 Grad und danach an der bewegten Umlenkrolle (11) in entgegengesetztem Sinn im Mittel um etwa 120 Grad umgelenkt wird, wobei der Winkel in der vorderen Stellung der Transfereinrichtung nicht weniger als etwa 100 Grad und in der hinteren Stellung der Transfereinrichtung nicht mehr als etwa 140 Grad beträgt.

## Claims

1. Process for the production of transdermal therapeutic systems, the layer containing active substance being present, initially, as a strip-shaped tape or web in the form of a laminate with a carrier layer and, possibly, further auxiliary and/or protective layers or the like, then being transferred either on its own or in combination with at least one further layer, in sections, to the centre of a second, wider sheet, characterized in that the strip-shaped tape or web (1) and the second web (2) are moved forward by steps, with different phases of motion and stationary or inoperative phases and/or different step lengths and/or differing speeds and that, in the same appliance, in phases, in the same forward direction, during each inoperative or stationary phase of the webs (1,2), the subdivision of the layer containing active substance and, possibly, of one or more further layers, with the exception of the carrier layer, takes place at right angles to the direction of the web, into sections (7,8) containing active substance of a size suitable for the user, and their transfer to the second web, the carrier sheet (9) being removed, in preferably equal intervals, using a transfer device moving backwards and forwards in the direction of the web.

2. Process according to Claim 1, characterized in that the transfer of the sections (7,8) containing active substance onto the second web (2) takes place during an inoperative or stationary phase of the webs, by means of the self-movement of a transfer device, known as such, with a dispenser edge (6).

3. Process according to Claim 1, characterized in that the transfer of the sections (7,8) containing active substance to the second web (2) during an inoperative or stationary phase of the webs (1,2) takes place by means of the self-movement of a transfer device designed as a laminating device with a laminating roll (12) and laminating table (13).

4. Process according to Claim 2, characterized in that the layer containing active substance in the strip-shaped tape or web (1) is positioned as a self-adhesive layer between an auxiliary layer and a sheet with an adhesive-repellent finish, and the sections (7) containing active substance, formed from the layer containing active substance and the auxiliary layer, are transferred to the second web (2), formed as an adhesive-repellent sheet, in such a way that the layer containing active substance lies directly on the sheet provided with the adhesive-repellent finish.

5. Process according to Claim 2 or 3, characterized in that the layer containing active substance in the strip-shaped tape or web is positioned as a self-adhesive or non-adhesive layer between an auxiliary layer and a carrier layer which is adhesive-repellent and that the sections containing active substance, formed from the layer containing active substance and the auxiliary layer, are transferred by means of the transfer device to the self-adhesive coated second web in such a way that the auxiliary layer lies directly on the self-adhesive sheet.

6. Process according to one of the Claims 1 to 3, characterized in that the layer containing active substance in the strip-shaped tape or web (1) is present as a laminate of three or more layers on a carrier sheet and that the sections (7) containing active substance are transferred to the second web (2) in the same order or the reciprocal order of the layers.

7. Process according to one of the Claims 1 to 6, characterized in that the layer containing active substance is applied to the carrier sheet as a non-adhesive coat.

8. Process according to Claims 1 to 7, characterized in that the movement of the transfer device and the movement of a clamp-like device (4) used for the forward movement of the first web (1) are coupled to one another.

9. Process according to one of the claims 1 to 8, characterized in that the transfer device, in addition to its movement in the direction of the web, is moved at right angles to the direction of the web and, during the inoperative or stationary phase of the webs (1,2), is pressed against the second web (2).

10. Device for executing the process according to one of the Claims 1 to 9, characterized in that for stripping the delaminated carrier sheet (9) of the first web (1), a first deflection or laminating roll (10 or 12, respectively), which can be moved together with the transfer device, and a second, fixed deflection roll (11) are provided, and that the stripped-off carrier sheet (9) on the transfer device is, initially, deflected by an angle of ca. 135 to 180 degrees and, thereafter, by the movable deflection roll (11) in the opposite direction, on average by about 120 degrees, the angle in the front position of the transfer device not amounting to less than ca. 100 degrees and in the rear position of the transfer device to not more than ca. 140 degrees.

## Revendications

1. Procédé pour la fabrication de systèmes thérapeutiques transdermiques, dans lequel la couche qui contient le produit actif se présentant tout d'abord en bande allongée, sous la forme d'un stratifié comportant une couche de support et éventuellement d'autres couches auxiliaires et/ou de protection ou similaires, et étant ensuite, seule ou en association avec au moins une autre couche, transférée par tronçons au milieu d'une seconde bande plus large, caractérisé en ce que la bande allongée (1) et la seconde bande (2) sont déplacées vers l'avant pas-à-pas dans des phases différentes de déplacement et de repos et/ou sur des longueurs d'avancement différentes et/ou à des vitesses différentes, et en ce que, chaque fois pendant la phase de repos des bandes (1, 2), on effectue de manière cadencée, dans le même dispositif, la division en tronçons de la couche qui contient le produit actif et éventuellement d'une ou plusieurs autres couches, à l'exception de la couche de support, transversalement par rapport à la direction de la bande, en tronçons (7, 8) qui contiennent le produit actif, à la taille d'utilisation, ainsi que le transfert sur la seconde bande, avec enlèvement de la feuille de support (9), de préférence aux mêmes écarts, au moyen d'un dispositif de transfert qui peut se déplacer en vaet-vient dans la direction de la bande.

2. Procédé selon la revendication 1, caractérisé en ce que le transfert des tronçons (7, 8) qui contiennent le produit actif sur la seconde bande (2) est effectué pendant une phase de repos des bandes, par le déplacement propre d'un dispositif de transfert en soi connu comportant un bord de distribution (6).

3. Procédé selon la revendication 1, caractérisé en ce que le transfert des tronçons (7, 8) qui contiennent le produit actif sur la seconde bande (2) s'effectue pendant une phase de repos des bandes (1, 2), par le déplacement propre d'un dispositif de transfert configuré comme dispositif de contre-collage présentant un rouleau de contre-collage (12) et une table de contre-collage (13).

4. Dispositif selon la revendication 2, caractérisé en ce que la couche qui contient le produit actif présente dans la bande allongée (1) est disposée comme couche autocollante entre une couche auxiliaire et une feuille équipée de manière à être non adhérente, et les tronçons (7) qui contiennent le produit actif, formés par la couche qui contient le produit actif et la couche auxiliaire, sont transférés au moyen du dispositif de transfert sur la seconde bande (2) configurée comme feuille non adhérente, de telle sorte que la couche qui contient le produit actif soit posée directement sur la feuille équipée de manière à être non adhérente.

5. Procédé selon la revendication 2 ou 3, caractérisé en ce que la couche qui contient le produit actif présente dans la bande allongée est disposée comme couche autocollante ou comme couche non collante entre une couche auxiliaire et une couche de support équipée de manière à être non adhérente, et les tronçons qui contiennent le produit actif, formés de la couche qui contient le produit actif et de la couche auxiliaire, sont transférés au moyen du dispositif de transfert sur la seconde bande enduite de manière à être autocollante, de telle sorte que la couche auxiliaire repose directement sur la feuille autocollante.

6. Procédé selon l'une des revendications 1 à 3, caractérisé en ce que la couche qui contient le produit actif présente dans la bande allongée (1) se présente comme stratifié constitué de trois ou de plus de trois couches sur une feuille de support, et les tronçons (7) qui contiennent le produit actif sont transférés sur la seconde bande (2) dans la même séquence des couches ou dans la séquence inverse.

7. Procédé selon l'une des revendications 1 à 6, caractérisé en ce que la couche qui contient le produit actif est appliquée comme couche non collante sur la feuille de support.

8. Procédé selon l'une des revendications 1 à 7, caractérisé en ce que le déplacement du dispositif de transfert et le déplacement d'un dispositif (4) en forme de pinces en vue du déplacement vers l'avant de la première bande (1) sont couplés l'un à l'autre.

9. Procédé selon l'une des revendications 1 à 8, caractérisé en ce qu'en plus de son déplacement dans la direction de la bande, le dispositif de transfert est déplacé perpendiculairement par rapport à la direction de la bande, et est repoussé contre la seconde bande (2) pendant la phase de repos des bandes (1, 2).

10. Dispositif pour la réalisation du procédé selon l'une des revendications 1 à 9, caractérisé en ce que pour enlever la feuille de support déstratifiée (9) de la première bande (1), il est prévu un premier rouleau de renvoi ou de contre-collage (10 ou 12) qui peut se déplacer avec le dispositif de transfert, et un second rouleau de renvoi fixe (11), et en ce que la feuille de support enlevée (9) est déviée sur le dispositif de transfert, tout d'abord d'un angle d'environ 135 à 180 degrés, et ensuite sur le rouleau de renvoi mobile (11), dans le sens opposé, en moyenne d'environ 120 degrés, tandis que, dans la position avant du dispositif de transfert l'angle n'est pas inférieur à environ 100 degrés, et qu'il n'est pas supérieur à environ 140 degrés dans la position arrière du dispositif de transfert.
